# EUROPEAN PATENT APPLICATION

(11) **EP 4 610 259 A1**
(43) Date of publication of application: **03.09.2025**
(21) Application number: 23881888.4
(22) Date of filing: 25.10.2023
(51) Int. Cl.: C07D 487/04, A61K 31/4985, A61K 31/5377, A61P 37/02, A61K 35/00, A61P 29/00, A61P 11/06

(54) **CRYSTAL FORM OF IMIDAZOPYRAZINE DERIVATIVE, PREPARATION METHOD THEREFOR AND USE THEREOF**

(30) Priority: 26.10.2022 CN 202211319172
(71) Applicant: TransThera Sciences (Nanjing), Inc., Nanjing, Jiangsu 210032 (CN)
(72) Inventor: DAI, Jianxin, Nanjing, Jiangsu 210032 (CN)
(74) Representative: Winter, Brandl - Partnerschaft mbB
(86) International application number: PCT/CN2023/126548
(87) International publication number: WO 2024/088311

(57) **Abstract**

The present invention belongs to the technical field of medicine, and specifically relates to a crystal form of an imidazopyrazine derivative represented by formula (I), a preparation method therefor and a use thereof.

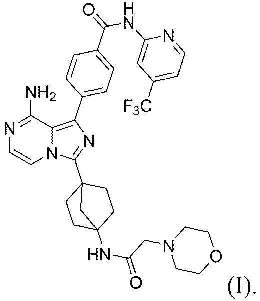

## Description

### TECHNICAL FIELD

The present disclosure pertains to the technical field of pharmaceuticals, and particularly relates to crystal forms of an imidazopyrazine derivative, a preparation method therefor, and use thereof.

### BACKGROUND

The B-cell receptor (BCR) is a transmembrane receptor located on the surface of B lymphocytes, and BCR signal transduction is crucial for normal B-cell development and acquired immunity. Abnormal BCR signal transduction can lead to dysregulation of B cell activation and/or the formation of pathogenic autoantibodies, resulting in various B-cell malignancies, autoimmune diseases, and inflammatory diseases. B-cell malignancies include chronic lymphocytic leukemia, small lymphocytic lymphoma, diffuse large B-cell lymphoma, follicular lymphoma, multiple myeloma, marginal zone lymphoma, mantle cell lymphoma, and Waldenstrom's macroglobulinemia. Autoimmune diseases and inflammatory diseases include rheumatoid arthritis, systemic lupus erythematosus, multiple sclerosis, and the like.

Bruton tyrosine kinase (BTK), a member of the non-receptor tyrosine kinase TEC family, is critical in the activation of the BCR signaling pathway. It is a key regulator of early B-cell formation as well as the activation and survival of mature B cells. BTK is important in regulating B-cell proliferation and apoptosis. Therefore, inhibition of BTK can be employed for treating tumors such as B-cell lymphoma and leukemia, as well as immune-related and inflammatory diseases.

The prior art WO2020063012A1 discloses an imidazopyrazine derivative as a BTK inhibitor, chemically named 4-(8-amino-3-(4-(2-morpholinoacetamido)bicyclo[2.2.1]heptan-1-yl)imidazo[1,5-a]pyrazin-1-yl)-*N*-(4-(trifluoromethyl)pyridin-2-yl)benzamide (hereinafter referred to as compound of formula (I)), having the following structural formula:

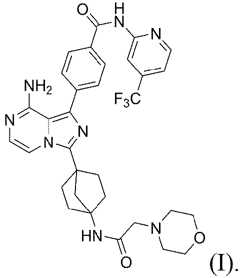

The prior art discloses its preparation method but does not disclose any crystal form information. In the research and development of medicaments, the study of crystal forms is very important. Different forms of a substance can lead to significant variations in physicochemical properties, biological activity, bioavailability, formulation, production, etc. The inventor of the present disclosure has conducted polymorphic studies on the compound to obtain an advantageous crystal form that is safe, effective, quality-controllable, stable, and conducive to pharmaceutical manufacturing.

### SUMMARY

In order to achieve the above objective, the present disclosure provides the following technical solutions:
The present disclosure provides a crystal form I of a compound of formula (I), which comprises characteristic peaks in an X-ray powder diffraction pattern using Cu-Kα radiation at 20 angles of 11.4±0.2°, 13.6±0.2°, 15.3±0.2°, 16.1±0.2°, 18.3±0.2°, and 20.7±0.2°;

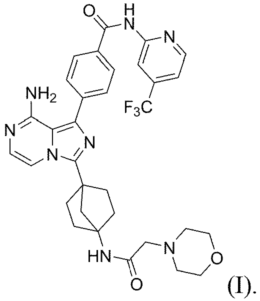

In some embodiments, the crystal form I further comprises characteristic peaks in an X-ray powder diffraction pattern using Cu-Kαradiation at 2θ angles of at least one of 12.3±0.2°, 19.4±0.2°, 19.9±0.2°, 21.2±0.2°, and 24.9±0.2°.

The present disclosure further provides a preparation method for the crystal form I of the compound of formula (I), which comprises the following steps:
dissolving the compound of formula (I) in diphenyl ether at 240 °C to 260 °C, and cooling to give the crystal form I.

In some embodiments, the diphenyl ether is used in an amount of 1-35 times the volume (e.g., 5-35 times the volume) of the compound of formula (I).

The present disclosure further provides a crystal form II of the compound of formula (I), which comprises characteristic peaks in an X-ray powder diffraction pattern using Cu-Kα radiation at 20 angles of 7.8±0.2°, 10.6±0.2°, 10.9±0.2°, 14.7±0.2°, 15.1±0.2°, and 19.7±0.2°;

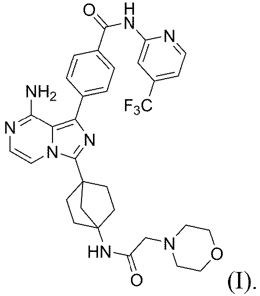

In some embodiments, the crystal form II further comprises characteristic peaks in an X-ray powder diffraction pattern using Cu-Kα radiation at 2θ angles of at least one of 12.7±0.2°, 17.0±0.2°, 18.0±0.2°, and 21.0±0.2°.

In some embodiments, the crystal form II further comprises characteristic peaks in an X-ray powder diffraction pattern using Cu-Kα radiation at 2θ angles of at least one of 23.0±0.2°, 23.6±0.2°, and 25.6±0.2°.

The present disclosure further provides a preparation method for the crystal form II of the compound represented by formula (I), which comprises the following steps:
placing the compound of formula (I) in a solvent, and performing a reflux reaction to give the crystal form II of the compound of formula (I), where the solvent is selected from one or more of a benzene solvent, an ether solvent, an ester solvent, and water.

In some embodiments, the preparation method for the crystal form II of the compound represented by formula (I) comprises the following steps:
placing the compound of formula (I) in an alcohol solvent, and reacting at a temperature range from 45 °C to the reflux temperature to give the crystal form II of the compound of formula (I).

In some embodiments, in the preparation method for the crystal form II, the alcohol solvent is selected from at least one of methanol, ethanol, isopropanol, and n-butanol; the benzene solvent is selected from toluene and/or dimethylbenzene; the ether solvent is selected from tetrahydrofuran and/or 2-methyltetrahydrofuran; the ester solvent is selected from ethyl acetate and/or isopropyl acetate.

In some embodiments, in the preparation method for the crystal form II, the solvent is selected from one or more of toluene, dimethylbenzene, tetrahydrofuran, 2-methyltetrahydrofuran, ethyl acetate, isopropyl acetate, an aqueous methanol solution, an aqueous ethanol solution, and an aqueous isopropanol solution; the alcohol solvent is selected from one or more of methanol, ethanol, isopropanol, and n-butanol.

In some embodiments, in the preparation method for the crystal form II, the solvent is selected from toluene, 2-methyltetrahydrofuran, an aqueous methanol solution, and ethyl acetate; the alcohol solvent is selected from methanol and ethanol.

In some embodiments, the aqueous methanol solution, the aqueous ethanol solution, and the aqueous isopropanol solution refer to mixed solutions of methanol, ethanol, and isopropanol with water, respectively.

In some embodiments, the volume ratio of alcohol to water in the aqueous methanol solution, the aqueous ethanol solution, and the aqueous isopropanol solution is greater than 1; preferably, the volume ratio of alcohol to water is greater than 1 and less than 100; preferably, the volume ratio of alcohol to water is greater than 4 and less than 100; preferably, the volume ratio of alcohol to water is greater than 30 and less than 100.

In some embodiments, the solvent is used in an amount of 1-35 times the volume (e.g., 5-35 times the volume) of the compound of formula (I).

In some embodiments, the reflux temperature refers to the boiling point temperature of the solvent used.

In some embodiments, the range from 45 °C to the reflux temperature may be 45 °C to 120 °C, or 50 °C to 120 °C, or 45 °C to 70 °C, or 45 °C to 85 °C.

In some embodiments, the alcohol solvent is selected from one or more of methanol, ethanol, isopropanol, and n-butanol.

In some embodiments, the alcohol solvent is selected from methanol.

In some embodiments, the alcohol solvent is used in an amount of 1-30 times the volume of the compound of formula (I).

The present disclosure further provides a crystal form III of the compound of formula (I), which comprises characteristic peaks in an X-ray powder diffraction pattern using Cu-Kα radiation at 20 angles of 5.9±0.2°, 10.4±0.2°, 11.8±0.2°, 15.7±0.2°, and 18.8±0.2°;

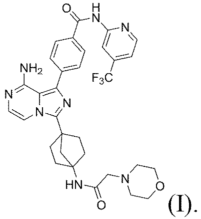

In some embodiments, the crystal form III further comprises characteristic peaks in an X-ray powder diffraction pattern using Cu-Kα radiation at 20 angles of at least one of 17.5±0.2°, 20.1±0.2°, and 21.4±0.2°.

The present disclosure further provides a preparation method for the crystal form III of the compound of formula (I), which comprises the following steps:
placing the compound of formula (I) in ethyl acetate at room temperature, and slurrying for 10 h to 36 h to give the crystal form III.

The present disclosure further provides a pharmaceutical composition comprising the crystal form I according to any one of the above, the crystal form II according to any one of the above, or the crystal form III according to any one of the above, and a pharmaceutically acceptable pharmaceutical carrier.

The present disclosure further provides a pharmaceutical formulation comprising the crystal form I according to any one of the above, the crystal form II according to any one of the above, or the crystal form III according to any one of the above, and a pharmaceutically acceptable pharmaceutical carrier.

In the embodiments of the present disclosure, the pharmaceutical composition and the pharmaceutical formulation may comprise one or more pharmaceutical carriers. The pharmaceutical carrier may be one or more solid or liquid fillers suitable for administration in humans. The pharmaceutical carrier preferably has sufficient purity and sufficiently low toxicity, and is compatible with the compound provided herein without significantly decreasing its efficacy. For example, the pharmaceutical carrier may be a filler, a binder, a disintegrant, a lubricant, an aqueous solvent, a non-aqueous solvent, or the like.

The pharmaceutical formulation described herein may be formulated into any pharmaceutically acceptable dosage form to administer a therapeutically effective amount of the aforementioned crystal form I, crystal form II, or crystal form III of the compound of formula (I) to a patient or a subject in need of such treatment in any suitable route of administration, such as oral, parenteral, rectal, or pulmonary administration. For oral administration, it may be formulated into tablets, capsules, pills, granules, and the like. For parenteral administration, it may be formulated into solution injections, sterile powders for injection, and the like.

The present disclosure further provides use of the crystal form I according to any one of the above, the crystal form II according to any one of the above, the crystal form III according to any one of the above, the pharmaceutical composition, or the pharmaceutical formulation in the manufacture of a medicament for preventing and/or treating B-cell malignancies, autoimmune diseases, and inflammatory diseases.

### Detailed Description of the Invention

The "room temperature" described herein refers to an indoor temperature. It usually is 10 °C to 30 °C, and may also be 15 °C to 25 °C.

The "times the volume" described herein refers to the volume (mL) of a solvent required to dissolve 1 g of a substance; for example, if 20 mL of a solvent is required to dissolve 1 g of a compound of formula (I), it is referred to as 20 times the volume.

The "therapeutically effective amount" described herein refers to an amount of the aforementioned crystal form I, II, or III of the compound of formula (I) contained in the composition or the pharmaceutical formulation that, when administered to a patient, is at least capable of alleviating symptoms of the patient's condition. An actual amount comprising the "therapeutically effective amount" will vary depending on a variety of circumstances, including, but not limited to, the particular condition being treated, the severity of the condition, the physique and health status of the patient, and the route of administration. The appropriate amount can be readily determined by skilled medical practitioners using methods known in the medical field.

### BENEFICIAL EFFECTS OF PRESENT INVENTION

Researches show that the crystal forms I, II, and III provided herein exhibit significantly improved solubility compared to the amorphous form. This enhances drug absorption in the human body, increases bioavailability, and thus improves the efficacy of the drug. Additionally, the higher solubility allows for a reduction in drug dosage while maintaining the therapeutic effect of the drug, thereby reducing the side effects of the drug and improving drug safety. Meanwhile, the crystal forms I, II, and III possess highly advantageous pharmacokinetic properties.

The present disclosure also discovers that the crystal forms I and II exhibit better stability under influencing factors, which can prevent the drug from being affected under non-label or harsh conditions. For example, during production, transportation, and storage, the drug may be exposed to high temperature, high humidity, or light due to seasonal and climatic variations, or weather factors. The improved stability ensures the therapeutic effect and safety of the drug.

The present disclosure also discovers that the crystal form II demonstrates low hygroscopicity, overcoming the drawbacks of highly hygroscopic active pharmaceutical ingredients, such as: weight changes due to moisture absorption, making it difficult to determine the component content of active pharmaceutical ingredients; tendency to absorb water and form lumps, affecting particle size distribution of samples in the formulation process and the uniformity of active pharmaceutical ingredients in the formulation, thereby affecting dissolution and bioavailability of active pharmaceutical ingredients; increased production costs due to the fact that highly hygroscopic active pharmaceutical ingredients have high requirements on storage conditions, packaging, and quality control.

The present disclosure also discovers that the crystal form II also exhibits good long-term stability, which is beneficial for drug storage.

### BRIEF DESCRIPTION OF THE DRAWINGS

FIG. 1 is an X-ray powder diffraction (XRPD) pattern of the amorphous form of the compound of formula (I).
FIG. 2 is a differential scanning calorimetry (DSC) pattern of the amorphous form of the compound of formula (I).
FIG. 3 is an X-ray powder diffraction (XRPD) pattern of the crystal form II of the compound of formula (I).
FIG. 4 is a differential scanning calorimetry (DSC) pattern of the crystal form II of the compound of formula (I).
FIG. 5 is an X-ray powder diffraction (XRPD) pattern of the crystal form I of the compound of formula (I).
FIG. 6 is a differential scanning calorimetry (DSC) pattern of the crystal form I of the compound of formula (I).
FIG. 7 is an X-ray powder diffraction (XRPD) pattern of the crystal form III of the compound of formula (I).
FIG. 8 is a differential scanning calorimetry (DSC) pattern of the crystal form III of the compound of formula (I).

### DETAILED DESCRIPTION

The above description of the present disclosure is further illustrated in detail below by way of specific embodiments, but it should not be construed that the scope of the above subject matter of the present disclosure is limited to the following examples. All techniques implemented based on the above description of the present disclosure fall within the scope of the present disclosure.

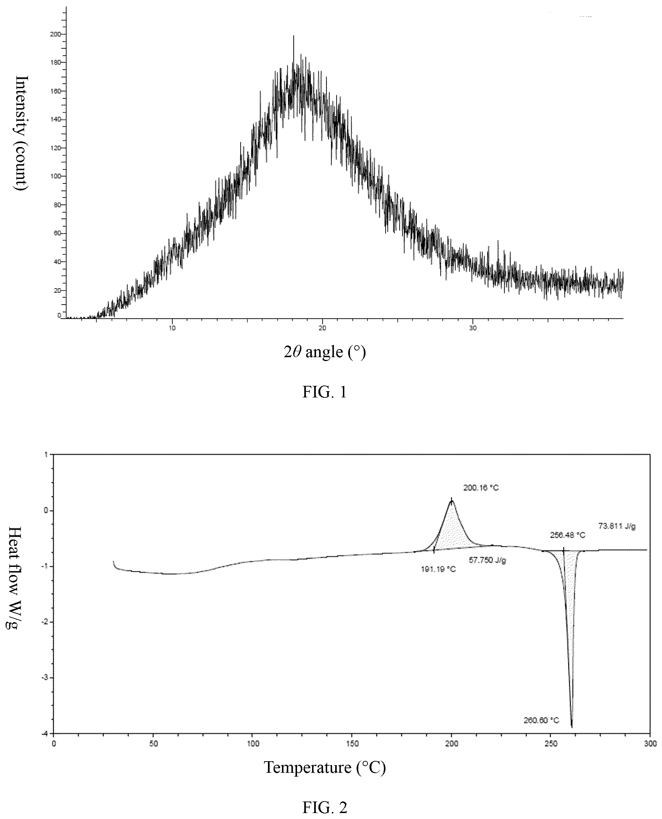

The compound of formula (I) used in the following examples was prepared according to the method described in WO2020063012A1. After testing, the compound was found to be in an amorphous form. The XRPD analysis is shown in FIG. 1, and the pattern obtained by differential scanning calorimetry (DSC) is shown in FIG. 2.

### Example 1: Preparation of crystal form II of compound of formula (I)

180 g of the compound of formula (I) was taken and dispersed in 270 mL of methanol. The mixture was heated to 50 °C and stirred, naturally cooled to room temperature, filtered under vacuum, and dried at 50 °C to give crystal form II.

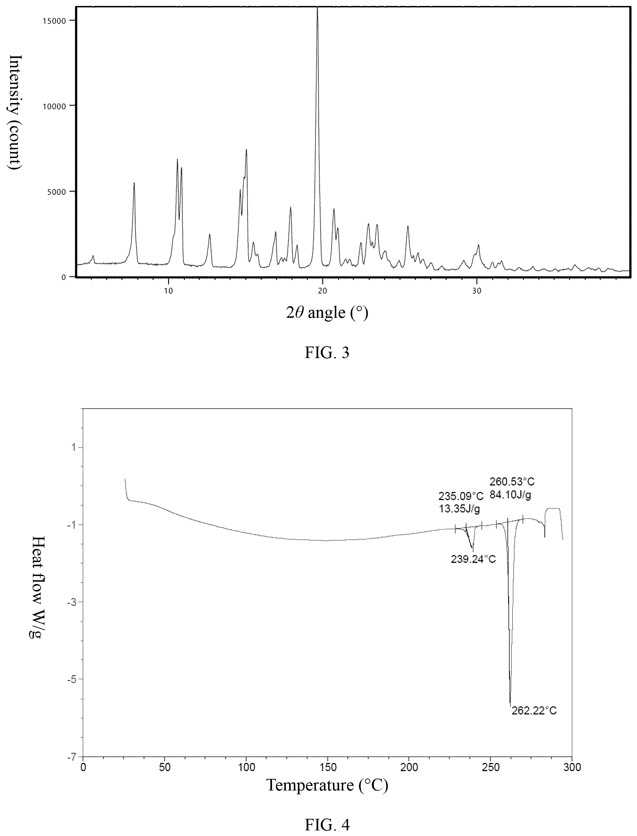

The XRPD analysis using Cu-Kα radiation is shown in FIG. 3.

As measured by a differential scanning calorimeter, the melting temperature of crystal form II was about 235 °C-239 °C, as shown in FIG. 4.

### Example 2: Preparation of crystal form II of compound of formula (I)

10 mL of 2-methyltetrahydrofuran was added to an eggplant-shaped flask and heated to reflux. Then, 0.96 g of the compound of formula (I) was added. After gradual dissolution, a solid was precipitated and filtered under vacuum to give crystal form II. The X-ray powder diffraction pattern using Cu-Kα radiation is substantially as shown in FIG. 3.

### Example 3: Preparation of crystal form II of compound of formula (I)

10 mL of toluene was added to an eggplant-shaped flask and heated to reflux. Then, 0.3 g of the compound of formula (I) was added. After gradual dissolution, a solid was precipitated and filtered under vacuum to give crystal form II. The X-ray powder diffraction pattern using Cu-Kα radiation is substantially as shown in FIG. 3.

### Example 4: Preparation of crystal form II of compound of formula (I)

10 mL of ethyl acetate was added to an eggplant-shaped flask and heated to reflux. Then, 0.4 g of the compound of formula (I) was added. After gradual dissolution, a solid was precipitated and filtered under vacuum to give crystal form II. The X-ray powder diffraction pattern using Cu-Kα radiation is substantially as shown in FIG. 3.

### Example 5: Preparation of crystal form II of compound of formula (I)

10 mL of ethanol was added to an eggplant-shaped flask and heated to reflux. Then, 0.8 g of the compound of formula (I) was added. After gradual dissolution, a solid was precipitated and filtered under vacuum to give crystal form II. The X-ray powder diffraction pattern using Cu-Kα radiation is substantially as shown in FIG. 3.

### Example 6: Preparation of crystal form II of compound of formula (I)

15 mL of methanol and 10 mL of water were added to an eggplant-shaped flask and heated to reflux. Then, 1.5 g of the compound of formula (I) was added. After gradual dissolution, a solid was precipitated and filtered under vacuum to give crystal form II. The X-ray powder diffraction pattern using Cu-Kα radiation is substantially as shown in FIG. 3.

### Example 7: Preparation of crystal form I of compound of formula (I)

15 mL of diphenyl ether was added to an eggplant-shaped flask and heated to 250 °C. Then, 2.5 g of the compound of formula (I) was added. A solid was precipitated during gradual dissolution and cooling and filtered under vacuum at 30 °C to give crystal form I.

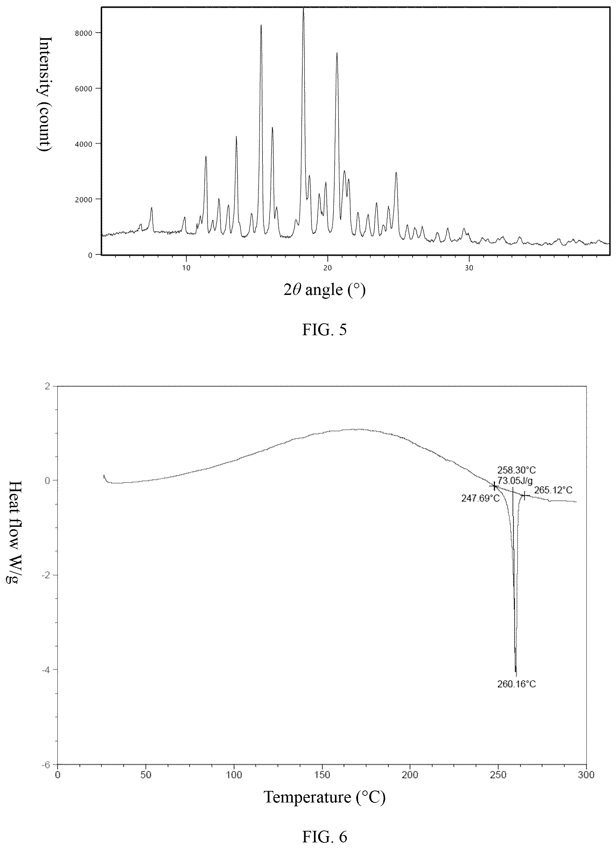

### The XRPD analysis using Cu-Kα radiation is shown in FIG. 5.

As measured by a differential scanning calorimeter, the melting temperature of crystal form I was about 258 °C-260 °C, as shown in FIG. 6.

### Example 8: Preparation of crystal form III of compound of formula (I)

10 g of the compound of formula (I) was taken and dispersed in 100 mL of ethyl acetate. The mixture was slurried at room temperature for about 16 h, filtered under vacuum, and dried in a forced air drying oven at 50 °C to give crystal form III.

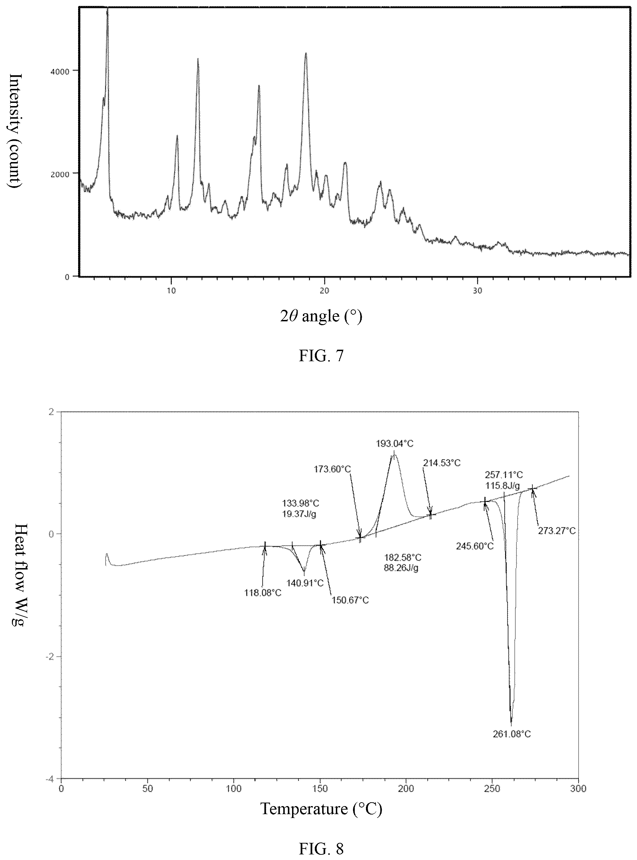

The XRPD analysis using Cu-Kα radiation is shown in FIG. 7.

As measured by a differential scanning calorimeter, the melting temperature of crystal form III was about 134 °C-141 °C, as shown in FIG. 8.

The present disclosure can be better understood according to the following experimental examples. However, it is easily understood by those skilled in the art that the contents described in the experimental examples are only used to illustrate the present disclosure, and should not and will not limit the present disclosure described in detail in the claims.

### Experimental Example 1: Solubility test of crystal forms I, II, III, and compound of formula (I) in pH 4.5 acetate buffer solution

Appropriate amounts of crystal forms I, II, III, and the compound of formula (I) were weighed and placed into 15 mL centrifuge tubes separately. A pH 4.5 acetate buffer was added to each tube to prepare saturated solutions. The tubes were sealed and placed in a water bath thermostatic oscillator to shake at 37 °C and a rotation speed of 200 rpm for 24 h. After centrifugation, the supernatants were collected and diluted to the corresponding multiples. The concentrations were measured. The detection was conducted according to the 2020 edition of the Chinese Pharmacopoeia. The results are shown in Table 1.

**Table 1. Solubility of crystal forms I, II, III, and compound of formula (I) in pH 4.5 acetate buffer solution**

| pH buffer | Solubility (µg/mL) | | | |
|---|---|---|---|---|
| | Crystal form I | Crystal form II | Crystal form III | Compound of formula (I) |
| pH4.5 | 273 | 375 | 98 | 65 |

The results indicated that crystal forms I, II, and III all exhibited higher solubility in pH 4.5 acetate buffer compared to the amorphous form.

### Experimental Example 2: Pharmacokinetic evaluation test of crystal forms I, II, and III in rats

### Animals: male SD rats

Animal administration and sample collection: Crystal forms I, II, and III were each dissolved in a mixture of 20% PEG400 + 80% (20% Captisol in 0.5% MC) to prepare administration formulation suspensions. The administration formulations were intragastrically administered to the SD rats at a dose of 100 mg/kg. Blood samples were collected at time points of 15 min, 30 min, 1 h, 2 h, 4 h, 6 h, 8 h, 24 h, 48 h, 72 h, and 96 h after administration.

One day before administration, the animals underwent jugular vein catheterization. After administration, about 300 µL of blood samples were collected via the jugular vein and then placed into anticoagulation tubes containing EDTA-K2. The blood samples were centrifuged at 4 °C and 8000 rpm for 10 min to yield plasma samples, which were prepared within 30 min after blood sample collection. The plasma samples were stored in a refrigerator at -80 °C before tests.

### Sample analysis method:

### Crystal form I and crystal form III:

The samples to be tested were taken out from the refrigerator at -80 °C, naturally thawed at room temperature, and then vortexed for 5 min. 20 µL of each of the 20-fold diluted plasma samples was precisely pipetted into a 1.5 mL centrifuge tube, and 300 µL of an internal standard working solution at a concentration of 10 ng/mL was added. The mixture was uniformly mixed, vortexed for 5 min, and then centrifuged at 12000 rpm for 5 min.

50 µL of the supernatant was precisely pipetted into a 96-well plate to which water was added at 150 µL/well in advance, uniformly mixed by vortexing for 5 min, and analyzed by LC-MS/MS.

### Crystal form II:

The sample to be tested was taken out from the refrigerator at -80 °C, naturally thawed at room temperature, and then vortexed for 5 min. 2 µL of the plasma sample was precisely pipetted and then added, together with 28 µL of blank plasma, to a 1.5 mL centrifuge tube, and 450 µL of an internal standard working solution (a solution of tolbutamide in acetonitrile) at a concentration of 100 ng/mL was added. The mixture was uniformly mixed, vortexed for 5 min, and then centrifuged at 12000 rpm for 5 min. 50 µL of the supernatant was precisely pipetted into a 96-well plate to which water was added at 150 µL/well in advance, uniformly mixed by vortexing for 5 min, and analyzed by LC-MS/MS.

### Data processing method:

The test compound concentrations were output by Analyst 1.7.1 of AB Sciex. Parameters such as mean, standard deviation, and coefficient of variation were calculated by Microsoft Excel. PK parameters were calculated by Pharsight Phoenix 8.2 software NCA (Tₘₐₓ was in median).

The results are shown in Table 2:

**Table 2. PK parameters of crystal forms I, II, and III in SD rats (p.o.: 100 mg/kg, mean, male, n = 3)**

| Crystal form | Administrat ion dosage (mg/kg) | t_{z1/2} (h) | Tₘₐₓ(h) | Cₘₐₓ (ng/mL) | AUCₗₐₛₜ (h*ng/mL) | AUC_{inf_obs} (h*ng/mL) |
|---|---|---|---|---|---|---|
| Crystal form I | 100 | 10.4 | 1.00 | 96267 | 1208628 | 1218508 |
| Crystal form II | 100 | 7.74 | 2.00 | 145250 | 2515536 | 2545383 |
| Crystal form III | 100 | 9.39 | 2.00 | 142333 | 2482493 | 2488128 |

| | | | | | | |
|---|---|---|---|---|---|---|
| Note: t_{z1/2}: terminal half-life; Tₘₐₓ: time to maximum plasma concentration; Cₘₐₓ: maximum plasma concentration; AUCₗₐₛₜ: area under plasma concentration-time curve from 0 to t; AUC_{inf_obs}: area under plasma concentration-time curve from 0 to infinity. | | | | | | |

As can be seen from Table 2, crystal forms I, II, and III all possess very good pharmacokinetic properties.

### Experimental Example 3: Stability evaluation under influencing factors

Appropriate amounts of crystal forms I, II, III, and the compound of formula (I) were taken and exposed to conditions of 60 °C, RH (relative humidity) 92.5%, RH (relative humidity) 75%, and light, respectively. The samples were collected on day 30, and the purity of the samples and changes in XRPD were examined according to the 2020 edition of the Chinese Pharmacopoeia. The results are shown in Table 3.

**Table 3. Examination results of crystal forms I, II, III, and compound of formula (I) under influencing factors for 30 days**

| Storage conditions | Purity % | Crystal form identification (XRPD) |
|---|---|---|
| 0 day | 99.3 | Crystal form II |
| 60 °C, 30 days | 99.3 | Consistent with day 0 |
| RH 92.5%, 30 days | 99.3 | Consistent with day 0 |
| RH 75%, 30 days | 99.3 | Consistent with day 0 |
| Light exposure, 30 days | 98.8 | Consistent with day 0 |
| 0 day | 98.6 | Crystal form I |
| 60 °C, 30 days | 98.7 | Consistent with day 0 |
| RH 92.5%, 30 days | 98.7 | Consistent with day 0 |
| RH 75%, 30 days | 98.7 | Consistent with day 0 |
| Light exposure, 30 days | 98.1 | Consistent with day 0 |
| 0 day | 99.0 | Crystal form III |
| 60 °C, 30 days | 94.8 | Consistent with day 0 |
| RH 92.5%, 30 days | 98.7 | Partially converted to amorphous |
| RH 75%, 30 days | 98.7 | Partially converted to amorphous |
| Light exposure, 30 days | 88.0 | Consistent with day 0 |
| 0 day | 99.7 | Compound of formula (I) |
| 60 °C, 30 days | 99.5 | Consistent with day 0 |
| RH 92.5%, 30 days | 99.7 | Consistent with day 0 |
| RH 75%, 30 days | 99.7 | Consistent with day 0 |
| Light exposure, 30 days | 88.2 | Consistent with day 0 |

As can be seen from Table 3, the purity of crystal forms I and II decreased by 0.5% under light exposure alone. After storage under all influencing factor conditions for 30 days, the measured XRPD pattern of crystal form I was substantially as shown in FIG. 5, and the measured XRPD pattern of crystal form II was substantially as shown in FIG. 3, indicating that no changes in the crystal forms occurred. The purity of crystal form III decreased by 11% under light exposure, and the purity decreased by 4.2% under the high temperature of 60 °C. The purity of the compound of formula (I) decreased by 11.5% under light exposure. Therefore, crystal forms I and II of the present disclosure exhibit higher stability compared to the amorphous form, making them more advantageous for pharmaceutical applications.

### Experimental Example 4: Long-term stability test

An appropriate amount of crystal form II was taken and packaged in low-density polyethylene bags with additional aluminum foil bags. The samples were stored under the conditions of 25 °C/60% RH (relative humidity) and 30 °C/65% RH (relative humidity), respectively, and then collected at 1M, 2M, 3M, 6M, 9M, 12M, and 18M. Additionally, the samples were stored under the conditions of 40 °C/75% RH (relative humidity) and then collected at 1M, 2M, 3M, and 6M. The appearance, total impurities, content, moisture, and crystal form changes (at 3M, 6M, and 12M) were examined and compared with the results on day 0. The results are shown in Table 4.

**Table 4. Long-term stability test results**

| Storage conditions | | Appearance | Total impurities % | Content % | Moisture % | Crystal form XRPD |
|---|---|---|---|---|---|---|
| 0 day | | Yellow solid | 1.1 | 98.3 | 0.23 | Crystal form II |
| 25°C | 1M | Yellow solid | 1.1 | 98.2 | 0.28 | NA |
| | 2M | Yellow solid | 1.2 | 98.3 | 0.16 | NA |
| | 3M | Yellow solid | 1.1 | 98.4 | 0.24 | Crystal form II |
| | 6M | Yellow solid | 1.1 | 98.2 | 0.16 | Crystal form II |
| | 9M | Yellow solid | 1.1 | 98.5 | 0.23 | NA |
| | 12M | Yellow solid | 1.1 | 98.2 | 0.12 | Crystal form II |
| | 18M | Yellow solid | 1.1 | 98.9 | 0.24 | NA |
| 30°C | 1M | Yellow solid | 1.1 | 98.2 | 0.24 | NA |
| | 2M | Yellow solid | 1.2 | 97.9 | 0.19 | NA |
| | 3M | Yellow solid | 1.1 | 98.2 | 0.30 | Crystal form II |
| | 6M | Yellow solid | 1.1 | 98.1 | 0.13 | Crystal form II |
| | 9M | Yellow solid | 1.1 | 98.4 | 0.24 | NA |
| | 12M | Yellow solid | 1.1 | 98.2 | 0.13 | Crystal form II |
| | 18M | Yellow solid | 1.1 | 99.0 | 0.17 | NA |
| 40°C | 1M | Yellow solid | 1.1 | 98.0 | 0.24 | NA |
| | 2M | Yellow solid | 1.2 | 97.9 | 0.18 | NA |
| | 3M | Yellow solid | 1.1 | 98.1 | 0.24 | Crystal form II |
| | 6M | Yellow solid | 1.1 | 98.3 | 0.13 | Crystal form II |

| | | | | | | |
|---|---|---|---|---|---|---|
| Note 1: NA indicates that it is not measured. | | | | | | |

Result analysis: Crystal form II showed no significant changes in appearance, total impurities, content, and moisture after being stored for 6 months under the conditions of 40 °C/75% RH and for 18 months under the conditions of 25 °C/60% RH and 30 °C/65% RH. The measured XRPD pattern was substantially as shown in FIG. 3, indicating that crystal form II exhibited good stability.

### Experimental Example 5: Hygroscopicity test

The experimental procedure was conducted in accordance with the guidelines for the hygroscopicity test of drugs in *Chinese Pharmacopoeia (2020 Edition).*
1. A dry glass weighing bottle with a stopper (outer diameter: 50 mm, height: 15 mm) was taken and placed in a thermostatic drier at 25 °C ± 1 °C (with a saturated ammonium chloride solution at the bottom) on the day before the test. After 24 h, the bottle was taken out, and its weight (m1) was precisely weighed.
2. An appropriate amount of crystal form II was taken and evenly spread in the weighing bottle described above to a thickness of about 1 mm. The total weight (m2) was precisely weighed.
3. The weighing bottle was left open, and both the bottle and its bottle cap were placed under the constant temperature and humidity conditions described above for 24 h.
4. The bottle was well sealed with its bottle cap, and the total weight (m3) was precisely weighed.
   Percentage weight gain (%) = 100% × (m3 - m2)/(m2 - m1)
5. Hygroscopicity characteristics and hygroscopic weight gains are defined as follows:
   deliquescent: sufficient water is absorbed to form a liquid;
   extremely hygroscopic: the hygroscopic weight gain is not less than 15%;
   hygroscopic: the hygroscopic weight gain is less than 15% but not less than 2%;
   slightly hygroscopic: the hygroscopic weight gain is less than 2% but not less than 0.2%;
   non-hygroscopic or almost non-hygroscopic: the hygroscopic weight gain is less than 0.2%.

**Table 5. Hygroscopicity of crystal form II**

| Name | m1(g) | m2(g) | m3(g) | 24 h percentage weight gain (%) |
|---|---|---|---|---|
| Crystal form II | 49.02882 | 50.04038 | 50.04381 | 0.34 |

According to the hygroscopicity test results, the 24 h percentage weight gain of crystal form II was 0.34% < 2%. Therefore, crystal form II is slightly hygroscopic, indicating low hygroscopicity.

The above description is only for the purpose of illustrating preferred examples of the present disclosure, and is not intended to limit the scope of the present disclosure. Any modifications, equivalents, improvements, and the like made without departing from the spirit and principle of the present disclosure should be included in the protection scope of the present disclosure.

## Claims

1. A crystal form I of a compound of formula (I), comprising characteristic peaks in an X-ray powder diffraction pattern using Cu-Kα radiation at 20 angles of 11.4±0.2°, 13.6±0.2°, 15.3±0.2°, 16.1±0.2°, 18.3±0.2°, and 20.7±0.2°;

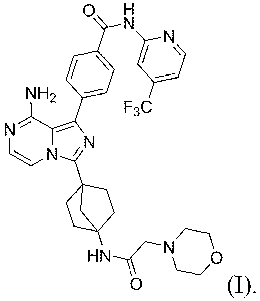

2. The crystal form I according to claim 1, further comprising characteristic peaks in an X-ray powder diffraction pattern using Cu-Kα radiation at 20 angles of at least one of 12.3±0.2°, 19.4±0.2°, 19.9±0.2°, 21.2±0.2°, and 24.9±0.2°.

3. A crystal form II of a compound of formula (I), comprising characteristic peaks in an X-ray powder diffraction pattern using Cu-Kα radiation at 20 angles of 7.8±0.2°, 10.6±0.2°, 10.9±0.2°, 14.7±0.2°, 15.1±0.2°, and 19.7±0.2°;

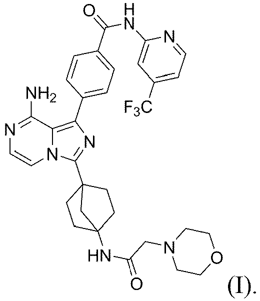

4. The crystal form II according to claim 3, further comprising characteristic peaks in an X-ray powder diffraction pattern using Cu-Kα radiation at 20 angles of at least one of 12.7±0.2°, 17.0±0.2°, 18.0±0.2°, and 21.0±0.2°.

5. The crystal form II according to claim 4, further comprising characteristic peaks in an X-ray powder diffraction pattern using Cu-Kα radiation at 20 angles of at least one of 23.0±0.2°, 23.6±0.2°, and 25.6±0.2°.

6. A preparation method for a crystal form II of a compound represented by formula (I), comprising the following steps:
placing the compound of formula (I) in a solvent, and performing a reflux reaction to give the crystal form II of the compound of formula (I), wherein the solvent is selected from one or more of a benzene solvent, an ether solvent, an ester solvent, and water; or
placing the compound of formula (I) in an alcohol solvent, and reacting at a temperature range from 45 °C to a reflux temperature to give the crystal form II of the compound of formula (I).

7. The preparation method for the crystal form II according to claim 6, wherein the alcohol solvent is selected from at least one of methanol, ethanol, isopropanol, and n-butanol; the benzene solvent is selected from toluene and/or dimethylbenzene; the ether solvent is selected from tetrahydrofuran and/or 2-methyltetrahydrofuran; the ester solvent is selected from ethyl acetate and/or isopropyl acetate.

8. The preparation method for the crystal form II according to claim 6, wherein the solvent is selected from one or more of toluene, dimethylbenzene, tetrahydrofuran, 2-methyltetrahydrofuran, ethyl acetate, isopropyl acetate, an aqueous methanol solution, an aqueous ethanol solution, and an aqueous isopropanol solution; the alcohol solvent is selected from one or more of methanol, ethanol, isopropanol, and n-butanol.

9. A crystal form III of a compound of formula (I), comprising characteristic peaks in an X-ray powder diffraction pattern using Cu-Kα radiation at 20 angles of 5.9±0.2°, 10.4±0.2°, 11.8±0.2°, 15.7±0.2°, and 18.8±0.2°;

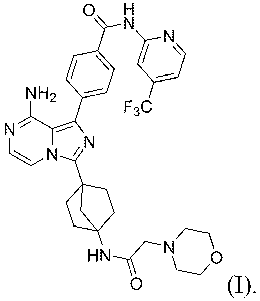

10. The crystal form III according to claim 9, further comprising characteristic peaks in an X-ray powder diffraction pattern using Cu-Kα radiation at 20 angles of at least one of 17.5±0.2°, 20.1±0.2°, and 21.4±0.2°.

11. A pharmaceutical composition, comprising the crystal form I according to claim 1 or 2, the crystal form II according to any one of claims 3-5, or the crystal form III according to claim 9 or 10, and a pharmaceutically acceptable pharmaceutical carrier.

12. A pharmaceutical formulation, comprising the crystal form I according to claim 1 or 2, the crystal form II according to any one of claims 3-5, or the crystal form III according to claim 9 or 10, and a pharmaceutically acceptable pharmaceutical carrier.

13. Use of the crystal form I according to claim 1 or 2, the crystal form II according to any one of claims 3-5, the crystal form III according to claim 9 or 10, the pharmaceutical composition according to claim 11, or the pharmaceutical formulation according to claim 12 in the manufacture of a medicament for preventing and/or treating B-cell malignancies, autoimmune diseases, and inflammatory diseases.
